# EUROPEAN PATENT SPECIFICATION

(11) **EP 2 298 396 B1**
(45) Date of publication and mention of the grant of the patent: **16.08.2017**
(21) Application number: 10176642.6
(22) Date of filing: 14.09.2010
(51) Int. Cl.: A61M 5/24, A61M 5/32

(54) **Hub assembly having a hidden needle for a drug delivery pen**
Hebeanordnung mit einer versteckten Nadel für einen Arzneimittelverabreichungsstift
Ensemble de moyeu doté d'une aiguille cachée pour stylo d'administration de médicaments

(30) Priority: 18.09.2009 US 563094
(43) Date of publication of application: 23.03.2011
(60) Divisional application: 11191452.9
(73) Proprietor: Becton, Dickinson and Company, Franklin Lakes, NJ 07417-1880 (US)
(72) Inventor: Horvath, Joshua D., Sparta, NJ 07871 (US); Andreoni, Todd, Lyndhurst, NJ 07071 (US); Gibney, Michael, Chestnut Ridge, NY 10977 (US); Tuttle, Ryan M., Morristown, NJ 07960 (US)
(74) Representative: dompatent von Kreisler Selting Werner - Partnerschaft von Patent- und Rechtsanwälten mbB

(56) References cited:
- WO-A1-02/20074
- DE-C1- 19 856 167
- US-A- 5 015 240
- US-A- 5 658 256

## Description

The present invention relates to a hidden needle for a pen injection device. More particularly, the present invention relates to a compressible member connected to a hub of the pen injection device that covers the needle to prevent a patient from seeing the needle, and compresses to expose the needle during the injection.

Medication delivery pens are hypodermic syringes used for self-injection of precisely measured doses of medication. Pens are widely used, for example, by diabetics to dispense insulin. A pen needle comprising a hub assembly as defined in the preamble of claim 1 is disclosed in WO 02/20074 A1. A typical prior art medication delivery pen includes a cartridge that contains a volume of liquid medication sufficient for several doses. The dose is into a tissue area, such as the intramuscular tissue layer, the subcutaneous tissue layer, or the intradermal tissue layer. The assembly and operation of a typical pen injection device, as shown in FIGS. 1 and 2, is described in US 2006/0229562 A1.

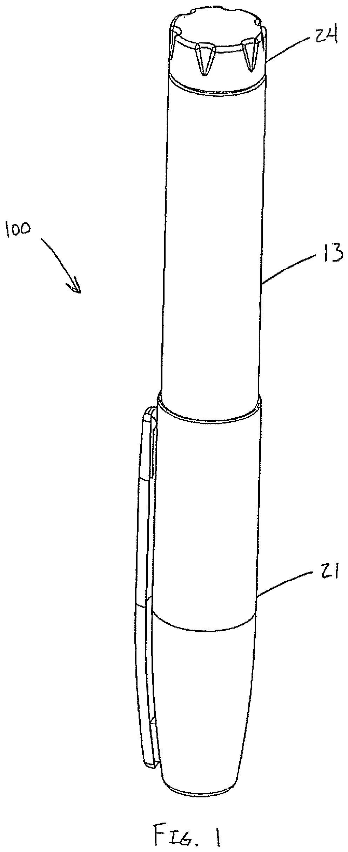

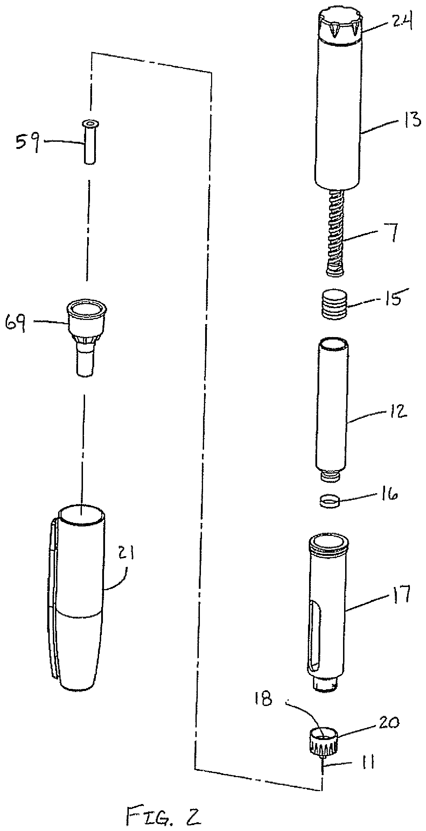

Pen injection devices, such as the exemplary pen injector 100, as shown in FIGS. 1 and 2, typically comprise a dose knob/button 24, an outer sleeve 13, and a cap 21. The dose knob/button 24 allows a user to set the dosage of medication to be injected. The outer sleeve 13 is gripped by the user when injecting medication. The cap 21 is used by the user to securely hold the pen injector device 100 in a shirt pocket, purse or other suitable location.

FIG. 2 is an exploded view of an exemplary drug delivery pen shown in FIG 1 The dose knob/button 24 has a dual purpose and is used to both set the dosage of the medication to be injected and to inject the dosed medicament via the lead screw 7 and stopper 15 through the medicament cartridge 12, which is attached to the drug delivery pen through a lower housing 17. In standard drug delivery pens, the dosing and delivery mechanisms are all found within the outer sleeve 13 and are not described in greater detail here as they are understood by those knowledgeable of the prior art The distal movement of the plunger or stopper 15 within the medicament cartridge 12 causes medication to be forced into the needle 11 of the hub 20. The medicament cartridge 12 is sealed by septum 16, which is punctured by a septum penetrating needle cannula 18 located within the hub 20. The hub 20 is preferably screwed onto the lower housing 17, although other attachment means can be used such as attaching to the cartridge. To protect a user, or anyone who handles the pen injection device 100, an outer shield 69, which attaches to the hub 20, covers the hub An inner shield 59 covers the patient needle 11 within the outer shield 69 The inner shield 59 can be secured to the hub 20 to cover the patient needle 11 by any suitable means, such as an interference fit or a snap fit. The outer shield 69 and inner shield 59 are removed prior to use. The cap 21 fits snugly against outer sleeve 13 to allow a user to securely carry the drug delivery pen 100.

The medicament cartridge 12 is typically a glass tube sealed at one end with the septum 16 and sealed at the other end with the stopper 15. The septum 16 is pierceable by a septum penetrating cannula 18, but does not move with respect to the medicament cartridge 12. The stopper 15 is axially displaceable within the medicament cartridge 12 while maintaining a fluid tight seal.

The outer shield 69 and the inner shield 59 are removed from the hub 20 and needle 11 prior to injecting a patient with the medicament stored in the cartridge 12. Some patients become uncomfortable at the sight of the needle 11, which is visible prior to the injection.

A hub assembly generally corresponding to the first part of claim 1 is disclosed in WO 02/20074 A1. The hub assembly comprises a locking device surrounding a needle supported by a needle hub. The locking device comprises a plurality of locking elements that are concentrically connected to each other. The locking elements act as a needle shield, which covers the needle after the needle has been used so that the needle is no longer accessible for a user after the first use. The needle protection device is surrounded by a bellow that is connected to most distal locking element. The bellow forms a needle sleeve and covering the needle in a protective position. The bellow is not a compressible member since it is only extendable and then locked in the extended position by the locking device.

A compressible member connected to a needle hub is disclosed in US 5,015,240. A safety retractable sheath covers an injection needle for protection against accidental pricking. The sheath extends from a base secured to the hub of the needle and has bellowed walls made of resilient material. It may be axially retracted towards the hub to expose the needle, and springs back to cover its entire length when released.

It is an object of the invention to provide a hub assembly for a pen injection device, which prevents a patient from seeing the needle prior to an injection.

The hub assembly of the present invention is defined by claim 1.

In accordance with an aspect of the present invention, a hub assembly for a pen injection device has a compressible member to prevent a patient from seeing the needle prior to an injection.

In accordance with another aspect of the present invention, the compressible member allows the needle to be primed without being seen by the patient.

The hub assembly for a pen injection device according to an exemplary embodiment of the present invention prevents a user from seeing the needle prior to the injection. A hub is connected to the pen injection device. A needle is received by the hub. A compressible member is connected to the hub and is movable between a first position that entirely covers the needle and a second position that exposes the needle for an injection.

Objects, advantages, and salient features of the invention will become apparent from the following detailed description, which, taken in conjunction with the annexed drawings, discloses exemplary embodiments of the invention.

The above benefits and other advantages of the various embodiments of the present invention will be more apparent from the following detailed description of exemplary embodiments of the present invention and from the accompanying figures, in which:
FIG. 1 is a perspective view of an assembled existing pen needle assembly;
FIG. 2 is an exploded perspective view of the components of the pen needle assembly of FIG. 1;
FIG. 3 is a perspective view of a hub assembly according to an exemplary embodiment of the present invention having a compressible member in a position covering a needle;
FIG. 4 is a perspective view of the hub assembly of FIG. 3 in which the compressible member is compressed to expose the needle;
FIG. 5 is a side elevational view of a hub assembly according to another exemplary embodiment not of the present invention having a compressible member in a position covering a needle; and
FIG. 6 is a side elevational view of the hub assembly of FIG. 5 in which the compressible member is compressed to expose the needle.

Throughout the drawings, like reference numbers will be understood to refer to like parts, components and structures.

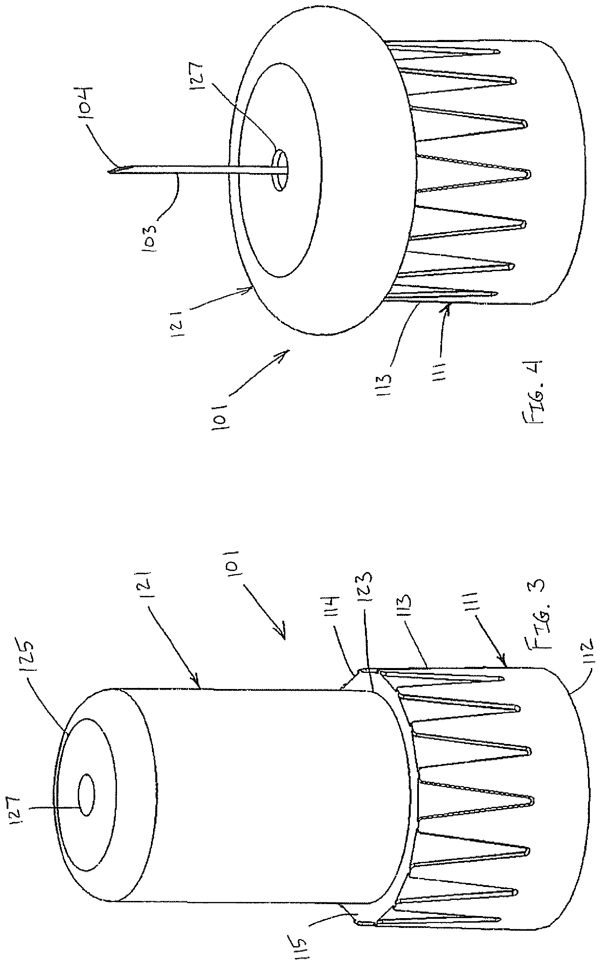

In an exemplary embodiment of the present invention, as shown in FIGS. 3 and 4, a hub assembly 101 for a pen injection device 100 (FIG. 2) prevents a patient from seeing a needle 103 of the hub assembly during the injection. A hub 111 of the hub assembly 101 is connected to the pen injection device. The needle 103 is received by the hub 111. A compressible member 121 is connected to the hub 111 such that the needle 103 is not visible during the injection.

The hub 111 is a typical hub, such as is shown in FIG. 2. The hub 111 has a body 113, which preferably has a cylindrical shape, having a first end 112 and a second end 114. A substantially planar base 115 is formed at the second end 114 of the hub body 113.

The compressible member 121 is disposed on the hub 111. An adhesive secures the first end 123 of the compressible member to the base 115 of the hub 111. The compressible member 121 has a preferably substantially cylindrical shape. An opening 127 at a second end 125 of the compressible member 121 allows the needle 103 to pass therethrough during an injection. Preferably, the compressible member 121 is a sponge made of a soft foam material.

The compressible member 121 is compressible between a first position, as shown in FIG. 3, in which the compressible member 121 is not compressed and covers substantially the entirety of the needle 103 and a second position, as shown in FIG. 4, in which the compressible member is compressed to expose the patient end 104 of the needle. Applying downward pressure on the pen injection device 100 (FIGS. 1 and 2) during an injection causes the compressible member 121 to compress, as shown in FIG. 4. Accordingly, the patient end 104 of the needle 103 is exposed such that medicament can be administered to the patient. After the medicament has been administered, the needle is removed such that pressure is no longer being applied to the compressible member. Accordingly, the compressible member 121 returns to the non-compressed position, as shown in FIG. 3.

The compressible member 121 may include a chemical indicator that changes the color of the compressible member when the needle 103 is primed. The medicament interacts with the chemical indicator, thereby causing a color change in the compressible member 121 to indicate priming of the needle. Accordingly, the needle 103 can be primed without the needle being seen by the patient.

The compressible member 121 may include adhesive microspheres. When the compressible member 121 is compressed, as shown in FIG. 4, the adhesive microspheres rupture. When the compressible member 121 moves back to the initial position, as shown in FIG. 3, the adhesive microspheres cure, thereby hardening the compressible member so that it is no longer compressible.

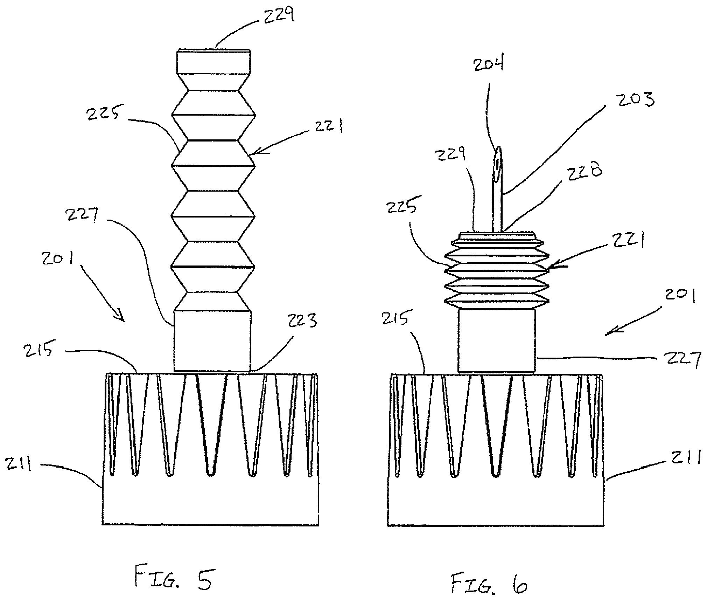

An exemplary embodiment of a hub assembly 201 not of the present invention is shown in FIGS. 5 and 6. The compressible member 221 is disposed on the hub 211.

Preferably, a friction fit between the hub post and the compressible 221 member secures the compressible member to the hub 211. Alternatively, an adhesive may be used to secure a first end 223 of the compressible member 221 to the base 215 of the hub 211. The compressible member 221 has a compressible bellows portion 225 and a non-compressible portion 227. An opening 228 at a second end 229 of the compressible member 221 allows the needle 203 to pass therethrough during an injection.

The compressible member 221 is movable between a first position, as shown in FIG 5, in which the compressible member 221 is not compressed and covers substantially the entirety of the needle 203 and a second position, as shown in FIG. 6, in which the compressible member is compressed to expose the patient end 204 of the needle. Applying downward pressure on the pen injection device 100 (FIGS. 1 and 2) during an injection causes the bellows portion 225 of the compressible member 221 to compress, as shown in FIG. 5. Accordingly, the patient end 204 of the needle 203 is exposed such that medicament can be administered to the patient. After the medicament has been administered, the needle is removed such that pressure is no longer being applied to the compressible member 221. Accordingly, the bellows portion 225 of the compressible member 221 returns to the non-compressed position, as shown in FIG. 5.

The foregoing embodiments and advantages are merely exemplary and are not to be construed as limiting the scope of the present invention. The description of exemplary embodiments of the present invention is intended to be illustrative, and not to limit the scope of the present intention. Various modifications, alternatives and variations will be apparent to those of ordinary skill in the art, and are intended to fall within the scope of the invention as defined in the appended claims and their equivalents.

## Claims

1. A hub assembly for a pen injection device, comprising:
a hub (111) for connecting to the pen injection device (100);
a needle (103) received by said hub; and
a compressible member (121) connected to said hub and compressible between a first position that entirely covers and prevents visibility of said needle and a second position that exposes the needle for an injection, said compressible member being formed as a one-piece member having an opening extending from a first end to a second end thereof through which said needle passes when said compressible member is in said second position,
**characterized in that**
said compressible member (121) is made of a foam material,
changes color when contacted by medicament during priming of said needle and
has an outer diameter small than an outer diameter of said hub (111) when said compressible member is in said first position.

2. The hub assembly of claim 1, wherein
an adhesive secures said compressible member to said hub.

3. The hub assembly of claim 1, wherein
said compressible member (121) has adhesive microspheres that rupture when said compressible member is moved to said second position such that said compressible member cures when returned to said first position.

## Patentansprüche

1. Ansatzvorrichtung für eine Pen-Injektionsvorrichtung, mit:
einem Ansatz (111) zur Verbindung mit der Pen-Injektionsvorrichtung (100);
einer von dem Ansatz aufgenommenen Nadel (103); und
einem zusammendrückbaren Teil (121), das mit dem Ansatz verbunden ist und zwischen einer ersten Position, in der die Nadel vollständig abgedeckt ist und eine Sicht auf die Nadel verhindert ist, und einer zweiten Position zusammendrückbar ist, in der die Nadel zur Injektion exponiert ist, wobei das zusammendrückbare Teil als einstückiges Teil mit einer sich von einem ersten Ende zu einem zweiten Ende des Teils erstreckenden Öffnung ausgebildet ist, durch welche die Nadel hindurchtritt, wenn sich das zusammendrückbare Teil in der zweiten Position befindet,
**dadurch gekennzeichnet, dass**
das zusammendrückbare Teil (121) aus einem Schaumstoffmaterial gebildet ist,
seine Farbe verändert, wenn es während der Erstbefüllung der Nadel von einem Medikament kontaktiert wird, und
einen Außendurchmesser hat, der kleiner als ein Außendurchmesser des Ansatzes (111) ist, wenn sich das zusammendrückbare Teil in der ersten Position befindet.

2. Ansatzvorrichtung nach Anspruch 1, bei dem ein Kleber das zusammendrückbare Teil an dem Ansatz sichert.

3. Ansatzvorrichtung nach Anspruch 1, bei dem das zusammendrückbare Teil (121) adhäsive Mikrokugeln aufweist, die bei Bewegung des zusammendrückbaren Teils in die zweite Position brechen, derart, dass das zusammendrückbare Teil nach der Rückkehr in die erste Position aushärtet.

## Revendications

1. Ensemble raccord pour un dispositif de type stylo injecteur, comprenant :
un raccord (111) pour se relier au dispositif de type stylo injecteur (100) ;
une aiguille (103) reçue par ledit raccord ; et
un élément compressible (121) relié audit raccord et compressible entre une première position qui recouvre entièrement ladite aiguille et empêche sa visibilité et une deuxième position qui expose l'aiguille pour une injection, ledit élément compressible étant formé en un élément monobloc ayant une ouverture s'étendant d'une première extrémité à une deuxième extrémité de celui-ci à travers laquelle ladite aiguille passe lorsque ledit élément compressible se trouve dans ladite deuxième position,
**caractérisé en ce que**
ledit élément compressible (121) est réalisé en un matériau en mousse, change de couleur lorsqu'il est mis en contact avec un médicament pendant l'amorçage de ladite aiguille et a un diamètre extérieur inférieur à un diamètre extérieur dudit raccord (111) lorsque ledit élément compressible se trouve dans ladite première position.

2. Ensemble raccord de la revendication 1, dans lequel
un adhésif fixe ledit élément compressible audit raccord.

3. Ensemble raccord de la revendication 1, dans lequel
ledit élément compressible (121) a des microsphères adhésives qui se rompent lorsque ledit élément compressible se déplace vers ladite deuxième position de sorte que ledit élément compressible durcit lorsqu'il retourne vers ladite première position.
